# EUROPÄISCHE PATENTSCHRIFT

(11) **EP 3 544 551 B1**
(45) Veröffentlichungstag und Bekanntmachung des Hinweises auf die Patenterteilung: **16.04.2025**
(21) Anmeldenummer: 17801437.9
(22) Anmeldetag: 15.11.2017
(51) Int. Cl.: A61F 2/38, A61F 2/30

(54) **ZWISCHENSEGMENT FÜR EINE GELENKKOMPONENTE**
INTERMEDIATE SEGMENT FOR A JOINT COMPONENT
SEGMENT INTERMÉDIAIRE POUR UN COMPOSANT D'ARTICULATION

(30) Priorität: 24.11.2016 DE 102016223289
(43) Veröffentlichungstag der Anmeldung: 02.10.2019
(73) Patentinhaber: Waldemar Link GmbH & Co. KG, 22339 Hamburg (DE)
(72) Erfinder: DMUSCHEWSKY, Klaus, 22397 Hamburg (DE)
(74) Vertreter: Alatis
(86) Internationale Anmeldenummer: PCT/EP2017/079301
(87) Internationale Veröffentlichungsnummer: WO 2018/095778

(56) Entgegenhaltungen:
- EP-A2- 0 850 606
- EP-A2- 0 850 608
- US-A- 5 571 194
- US-A1- 2005 075 736
- US-A1- 2014 081 408
- US-A1- 2015 335 438

## Beschreibung

### GEBIET DER ERFINDUNG

Die Erfindung betrifft ein Zwischensegment für eine Anordnung zwischen einer konkaven Implantationsfläche einer Gelenkkomponente und Knochengewebe, um fehlendes Gewebe zum Unterstützen der Prothese zu ersetzen. Weiterhin betrifft die Erfindung eine Gelenkkomponente mit einem Zwischensegment und einem Verfahren zum Montieren und Befestigen eines Zwischensegments an einer Gelenkkomponente.

### STAND DER TECHNIK

Bei der Implantation eines künstlichen Gelenkersatzes kann es vorkommen, dass mehr vom Knochenstock eines Patienten entfernt werden muss oder bereits fehlt, als für ein korrektes Einpassen einer Gelenkkomponente des Gelenkersatzes notwendig ist. Folglich existiert in so einer Situation zumindest abschnittsweise ein Leerraum zwischen der Gelenkkomponente und dem Knochengewebe. Ursächlich hierfür kann eine bereits vorliegende Schädigung des Knochengewebes sein, wie zum Beispiel durch eine Fraktur, einen Tumor oder Nekrose. Die Folge ist, dass die Seite oder Fläche der Gelenkkomponente, die für ein Befestigen dieser an dem Knochengewebe vorgesehen ist, nicht mehr über deren ganze Erstreckung optimal zu dem Knochengewebe angeordnet werden kann.

Für den Fall, dass es sich bei der dem Knochengewebe zugewandten Seite der Gelenkkomponente um eine konvexe Seite handelt, wie zum Beispiel bei einer Gelenkpfanne eines Hüftgelenkersatzes der Fall, ist es möglich, vor oder auch bei Implantation Knochengewebe aufzubauen und trotzdem relativ schnell eine stabile Verankerung zu erreichen. Einfach gesagt wird eine zu große Aushöhlung des Knochengewebes vor Implantation aufgefüllt und dann mit der Gelenkkomponente "verschlossen." Bei dieser Anordnung ist es von Vorteil, dass die Versorgung des Knochengewebes im Vergleich zu dem Fall günstiger ist, bei dem eine konkave Implantationsseite einer Gelenkkomponente das Knochengewebe nach Implantation zumindest teilweise umschließt. Bei Letzterem wird die Versorgung des Knochens verschlechtert. Zudem kann bei einer teilweisen Umschließung der Knochen nach außen hin offen liegen, sodass zusätzliche Maßnahmen notwendig sind, um das zum Auffüttern des Knochens verwendete Material einzudämmen. Als Alternative werden die genannten Leerräume deswegen häufig einfach mit Knochenzement ausgefüllt. Als Ergebnis ist eine Befestigung der Gelenkkomponente über Knocheneinwuchs nicht mehr möglich. Auch wenn die Gelenkkomponente von Anfang an für eine Verankerung über Knochenzement vorgesehen war, ergibt sich durch den aufzufüllenden Bereich ein teilweise erheblich größeres Volumen an Knochenzement, das notwendig ist, um diesen Bereich aufzufüllen. Hierdurch besteht die Gefahr, dass das umliegende Knochengewebe einer im Vergleich höheren Temperatur und dieser zudem über einen längeren Zeitraum ausgesetzt wird. Da sich das Ausmaß fehlenden Knochengewebes zumeist erst während der Operation abschätzen lässt, ist auch eine individuell an den Patienten angepasste Gelenkkomponente keine gute und zudem eine sehr kostenintensive Alternative.

In diesem Zusammenhang offenbart die EP 0 850 608 A2, welche die Grundlage für die zweiteilige Form der unabhängigen Ansprüche bildet, einen Augmentationsblock. Dieser wird an der oberen Oberfläche eines Gelenkelements befestigt und hat eine Öffnung, die einen entsprechenden Fixationsstift umgibt. Das System umfasst ferner eine Druckhülse, die zwischen der Öffnung des Augmentationsblocks und dem Fixationsstift sitzt. Bei der Montage drückt eine über dem Fixationsstift angebrachte Sicherungsvorrichtung die Kompressionsspannzange zusammen.

Aufgabe der vorliegenden Erfindung war es somit, eine Möglichkeit bereitzustellen, mit der eine Gelenkkomponente in den oben genannten Fällen an den individuell vorhandenen Knochenstock eines Patienten angepasst werden kann, ohne das Knochengewebe weiter zu schädigen.

### ZUSAMMENFASSUNG DER ERFINDUNG

Als Lösung stellt die vorliegende Erfindung ein Zwischensegment für eine Anordnung zwischen einer konkaven Implantationsfläche einer Gelenkkomponente, insbesondere einer Gelenkprothese und bevorzugt einer Femurprothese, und Knochengewebe bereit. Das Zwischensegment weist einen Zwischensegmentkörper mit mindestens einer der Gelenkkomponente zugewandten Seite, wobei eine der mindestens einen der Gelenkkomponente, insbesondere Gelenkprothese, zugewandten Seite konvex ist, und einen Verriegelungsmechanismus zum Anbringen des Zwischensegments an der Gelenkkomponente, insbesondere Gelenkprothese, mit mindestens einem ersten und einem zweiten Rastelement auf, wobei mindestens eines der Rastelemente beweglich ist.

Das Zwischensegment ist ein von der Gelenkkomponente getrenntes Teil, das jedoch über die Rastelemente an der Gelenkkomponente mechanisch und vorzugsweise über einen Formschluss verriegelt werden kann. Dies hat den Vorteil, dass über eine Bereitstellung verschiedener Zwischensegmente das am besten passende Zwischensegment ausgewählt werden kann, um so die Gelenkkomponente an den Implantationsort anzupassen. Dabei ist es möglich, diese Anpassung während des chirurgischen Eingriffs vorzunehmen, sodass auch noch Anpassungen kurz vor Implantation der Gelenkkomponente durchführbar sind.

Die Verriegelung erfolgt über ein in Eingriff Bringen des mindestens einen beweglichen Rastelements mit der Gelenkkomponente. Sind sämtliche Rastelemente im Eingriff, ist das Zwischensegment fest an der Gelenkkomponente angebracht bzw. verriegelt.

Bei einer besonders bevorzugten Ausführungsform ist bei dem Zwischensegment das mindestens eine bewegliche Rastelement zwischen einer Freigabeposition und einer Verriegelungsposition bewegbar, wobei das bewegliche Rastelement in der Verriegelungsposition vorzugsweise von dem Zwischensegmentkörper hervorsteht.

Bei dieser Ausführungsform ist der Eingriff des beweglichen Rastelements reversibel, sodass das Zwischensegment bei Bedarf wieder von der Gelenkkomponente lösbar ist. Damit unterstützt auch diese Ausführungsform auf vorteilhafte Weise den modularen Einsatz der Zwischensegmente.

Die Ausführung, bei der das bewegliche Rastelement in der Verriegelungsposition von dem Zwischensegmentkörper hervorsteht, ist dabei insbesondere konstruktiv von Vorteil. So sind derartige Zwischensegmente auf einfache Weise bei vielfältigen und bereits bestehenden Gelenkkomponenten einsetzbar. Bei bereits bestehenden Gelenkkomponenten muss lediglich eine vorzugsweise komplementäre Aussparung für einen Eingriff des Rastelements eingebracht werden, um an einer solchen Gelenkkomponente Zwischensegmente anbringen zu können. Zudem ergibt sich bei der Vorbereitung des Implantationsorts der Vorteil, dass ohne Zwischensegment die für einen Kontakt mit der Gelenkkomponente vorgesehene Knochenfläche nicht mit einer Aussparung vorbereitet werden muss.

Bei einer weiteren besonders bevorzugten Ausführungsform ist zumindest eines der Rastelemente ein feststehendes Rastelement, wobei das feststehende Rastelement vorzugsweise von dem Zwischensegmentkörper hervorsteht.

Diese Ausführungsform ermöglicht eine besonders einfache Verriegelung eines Zwischensegments an der Gelenkkomponente, indem zunächst das feststehende Rastelement und nachfolgend das bewegliche Rastelement durch Einnahme einer Verriegelungsposition mit der Gelenkkomponente in Eingriff gebracht wird. Im Falle eines hervorstehenden feststehenden Rastelements werden die gleichen im Zusammenhang mit der vorigen Ausführungsform genannten Vorteile erreicht. Weiterhin ist das feststehende Rastelement vorzugsweise integral oder einstückig mit dem Zwischensegmentkörper ausgebildet und weist damit eine hohe strukturelle Festigkeit auf.

Bei einer weiteren Ausführungsform des Zwischensegments weist der Zwischensegmentkörper mindestens eine Verankerungsfläche zum Anbringen des Zwischensegmentkörpers an Knochengewebe auf. Dabei weist die Verankerungsfläche vorzugsweise eine Oberflächenstruktur und/oder eine Beschichtung auf, die noch bevorzugter für ein Einwachsen von Knochengewebe eingerichtet ist.

Bei dieser Ausführungsform ist eine Verankerungsfläche des Zwischensegmentkörpers vorzugsweise über ihre Oberflächenbeschaffenheit dazu eingerichtet, möglichst gute Bedingungen für eine Verbindung mit dem Knochengewebe eines Patienten bereitzustellen. Dies kann beispielsweise eine polierte Oberfläche sein, um eine Verbindung über Knochenzement herzustellen. Weiterhin kann die Verankerungsfläche durch eine raue oder poröse Oberfläche eine Verankerung der Gelenkkomponente über ein Einwachsen von Knochengewebe ermöglichen.

Durch die Verbindung mit dem Knochengewebe wird das Zwischensegment folglich insbesondere bei dieser Ausführungsform zu einem strukturell und funktionell integralen Teil der Gelenkkomponente. Weiterhin kann eine Oberflächenstruktur und/oder eine Beschichtung auch für den Einsatz von Knochenzement von Vorteil sein, da hierdurch die Kontaktfläche zum Knochenzement vergrößert und damit die Ableitung von Wärme verbessert wird.

Erfindungsgemäß weist das erste Rastelement eine erste Eingriffsachse und das zweite Rastelement eine zweite Eingriffsachse auf, wobei der Winkel zwischen der ersten Eingriffsachse und der zweiten Eingriffsachse ungleich null ist.

Aufgrund der in einem Winkel zueinander angeordneten Eingriffsachsen der mindestens zwei Rastelemente erfolgt durch das in Eingriff Bringen dieser Rastelemente ein Formschluss des Zwischensegments mit der Gelenkkomponente. Dabei ist unter einer Eingriffsachse die Achse zu verstehen, entlang der ein Rastelement bewegt wird, um mit einem gegenüberliegenden vorzugsweise komplementär ausgebildeten Eingriffselement der Gelenkkomponente in Eingriff zu gehen und so vorzugsweise einen Formschluss zwischen dem Zwischensegment und der Gelenkkomponente hervorzurufen. Weiterhin laufen die Eingriffsachsen der Rastelemente vorzugsweise von dem Zwischensegmentkörper aus auseinander.

Bei einer besonders bevorzugten Ausführungsform sind die Rastelemente zum Zusammenwirken mit der Gelenkkomponente an der konvexen Seite des Zwischensegments angeordnet.

Eine derartige Anordnung ist besonders vorteilhaft in Bezug auf die durch das künstliche Gelenk verlaufende Gelenkkraft, da diese das Zwischensegment und die Gelenkkomponente zusammendrückt und die Rastelemente bzw. Eingriffselemente so, wenn überhaupt, nur eine geringfügige Belastung erfahren. Da parallel zu den Gelenkflächen ebenfalls nur geringe durch Gelenkreibung verursachte Kräfte übertragen werden, kann auch eine Schubbelastung der Verbindung zwischen dem Zwischensegment und der Gelenkkomponente gering gehalten werden.

Bei einer weiteren besonders bevorzugten Ausführungsform ist das bewegliche Rastelement des Zwischensegments als Stift ausgebildet. Dieser Stift weist in seiner Längsrichtung auf einer Seite einen Verriegelungsabschnitt und auf der anderen gegenüberliegenden Seite vorzugsweise einen Werkzeugeingriffsabschnitt auf. Besonders bevorzugt ist der Stift als Gewindestift ausgebildet.

Das als Stift ausgebildete bewegliche Rastelement ist bevorzugt in der Längsrichtung des Stifts zwischen der Freigabeposition und der Verriegelungsposition bewegbar. Durch den Werkzeugeingriffsabschnitt kann dieses bewegliche Rastelement betätigt werden. Dabei erfolgt die Bewegung des beweglichen Rastelements über dessen Betätigung vorzugsweise über einen Gewindeeingriff zwischen dem Rastelement und dem Zwischensegmentkörper. Genauer gesagt erfolgt eine Bewegung des Stifts durch eine Drehung dieses Stifts über den Werkzeugeingriffsabschnitt. Diese Drehung wird über das Gewinde dieses Stifts in eine Längsbewegung umgewandelt. Ein derartig ausgebildetes bewegliches Rastelement weist einen besonders einfachen und widerstandsfähigen Aufbau auf. Zudem kann das Gewinde selbsthemmend sein, sodass sich das bewegliche Rastelement nicht von selbst löst.

Bei einer besonders bevorzugten Ausführungsform des Zwischensegments ist der Werkzeugeingriffsabschnitt des als Stift ausgebildeten beweglichen Rastelements für ein Bewegen des Stifts von einer freien Seite des Zwischensegmentkörpers aus zugänglich.

In diesem Zusammenhang ist unter einer freien Seite eine Seite des Zwischensegmentkörpers zu verstehen, die im implantierten Zustand der Gelenkkomponente nicht an Knochengewebe anliegt oder der Gelenkkomponente zugewandt ist. Eine solche Ausführung des Zwischensegments ist insbesondere im Falle einer Revision der Gelenkkomponente von Vorteil, da durch ein Lösen der Verriegelung des Zwischensegments bereits ein Teil der Gelenkkomponente von dem Knochengewebe getrennt wird und so die Gelenkkomponente leichter entfernt werden kann. Das Zwischensegment kann nach einem Austausch der Gelenkkomponente entweder wiederverwendet oder entfernt werden, wobei bei einer Entfernung durch die bereits abgenommene Gelenkkomponente ein sehr guter Zugang zu dem Zwischensegment ermöglicht wird.

Bei einer weiteren bevorzugten Ausführungsform weist der Zwischensegmentkörper einen bevorzugt als Aussparung ausgeführten Sichtabschnitt auf.

Dieser Sichtabschnitt vereinfacht das Verriegeln des Zwischensegments an der Gelenkkomponente und ist vorzugsweise auf der Seite des beweglichen Rastelements angeordnet, sodass während der Montage des Zwischensegments der Eingriff des beweglichen Rastelements mit einem Eingriffselement der Gelenkkomponente beobachte und überprüft werden kann. Mit anderen Worten ist beispielsweise bei einem sich für eine Verriegelungsposition aus dem Zwischensegmentkörper heraus bewegendes Rastelement die Eingriffsbewegung in die Gelenkkomponente durch den Sichtabschnitt sichtbar. Damit werden bei dieser Ausführungsform die Montage und eine Überprüfung des Verriegelungszustands erheblich erleichtert.

Des Weiteren stellt die vorliegende Erfindung eine Gelenkkomponente und bevorzugt eine Femurkomponente bereit. Diese Gelenkkomponente weist eine konkave Implantationsfläche mit einem über einen Verriegelungsmechanismus an der Gelenkkomponente befestigbaren Zwischensegment auf. Das Zwischensegment weist eine konvexe Seite auf, die im befestigten bzw. verriegelten Zustand der konkaven Implantationsfläche der Gelenkkomponente zugewandt ist.

Bei dieser Kombination aus einer Gelenkkomponente und einem Zwischensegment können die zuvor bereits erläuterten Vorteile für die Implantation und Revision der Gelenkkomponente an Knochengewebe eines Patienten erreicht werden.

Bei einer besonders bevorzugten Ausführungsform weist die Gelenkkomponente mindestens zwei Eingriffselemente auf, wobei die Eingriffselemente auf zumindest einer dem Zwischensegment zugewandten Seite, vorzugsweise der konkaven Implantationsfläche, angeordnet sind.

Die Eingriffselemente der Gelenkkomponente sind dabei vorzugsweise zumindest teilweise komplementär zu den Rastelementen des Zwischensegments ausgebildet. Wie oben bereits erwähnt, sind die Eingriffselemente bei der Gelenkkomponente vorzugsweise als Aussparungen ausgebildet.

Bei einer weiteren besonders bevorzugten Ausführungsform ist das Zwischensegment über einen Formschluss an der Gelenkkomponente befestigbar.

Mit anderen Worten erfolgt bei dieser Ausführungsform eine Verriegelung des Zwischensegments an der Gelenkkomponente durch einen Eingriff der Rastelemente und insbesondere des mindestens einen beweglichen Rastelements, sodass die Rastelemente einer Relativbewegung zwischen dem Zwischensegment und der Gelenkkomponente vorbeugen. Diese Verbindungsart hat die Vorteile, dass sie zum einen lösbar ist und zum anderen nicht von einer Reibung zwischen dem Zwischensegment und der Gelenkkomponente abhängig ist, wie bei einer Verriegelung über einen Reibschluss der Fall wäre.

Weiterhin stellt die vorliegende Erfindung ein Verfahren zum Befestigen eines Zwischensegments an einer konkaven Implantationsfläche einer Gelenkkomponente bereit, wobei das Verfahren die folgenden Schritte umfasst. Für die Montage des Zwischensegments an der Gelenkkomponente wird ein erstes Rastelement des Zwischensegments mit einem ersten Eingriffselement der Gelenkkomponente in Eingriff gebracht. Zudem wird ein zweites Rastelement des Zwischensegments mit einem zweiten Eingriffselement der Gelenkkomponente in Eingriff gebracht. Dabei ist mindestens eines der Rastelemente des Zwischensegments ein bewegliches Rastelement und die Eingriffsachsen der Rastelemente weisen einen Winkel auf, der ungleich null ist.

Durch dieses Verfahren kann auf einfache Weise, wie oben bereits im Zusammenhang mit dem Zwischensegment beschrieben, eine Gelenkkomponente vor Implantation modular an einen Implantationsort angepasst werden.

Bei einer besonders bevorzugten Ausführungsform erfolgt der Eingriff zwischen den Rastelementen des Zwischensegments und den Eingriffselementen der Gelenkkomponente über einen Formschluss an der konkaven Implantationsfläche der Gelenkkomponente, um dadurch die oben bereits beschriebenen Vorteile bei der Befestigung des Zwischensegments an der Gelenkkomponente zu erreichen.

### KURZE BESCHREIBUNG DER FIGUREN

Die folgenden Figuren veranschaulichen bevorzugte Ausführungsformen der vorliegenden Erfindung. Diese sind jedoch nicht dazu gedacht, den Schutzbereich der Ansprüche einzuschränken, sondern dienen zusammen mit der folgenden Beschreibung lediglich einem einfacheren Verständnis der Erfindung. In den Figuren beziehen sich gleiche Bezugszeichen auf Merkmale, welche die gleiche oder eine äquivalente Funktion und/oder Struktur aufweisen. Dabei veranschaulichen die Figuren Ausführungsformen des Zwischensegments und der Gelenkkomponente wie folgt:
Figur 1 ist eine dreidimensionale schematische Ansicht einer Gelenkkomponente, an der ein Zwischensegment befestigbar ist.
Figur 2a ist eine dreidimensionale schematische Ansicht eines Zwischensegments von der Seite aus, die im verriegelten Zustand des Zwischensegments der Gelenkkomponente zugewandt ist.
Figur 2b zeigt eine dreidimensionale schematische Ansicht eines Zwischensegments von der Seite aus, die im verriegelten Zustand des Zwischensegments dem Knochengewebe des Implantationsorts zugewandt ist.
Figur 3a zeigt die Gelenkkomponente aus Figur 1 von der Implantationsseite aus.
Figur 3b entspricht der Ansicht in Figur 3a mit einem implantierten Zwischensegment an der konkaven Fläche der Gelenkkomponente.
Figur 4 ist eine Schnittansicht entlang der Linie A-A aus Figur 3b.

### AUSFÜHRLICHE BESCHREIBUNG BEVORZUGTER AUSFÜHRUNGSFORMEN

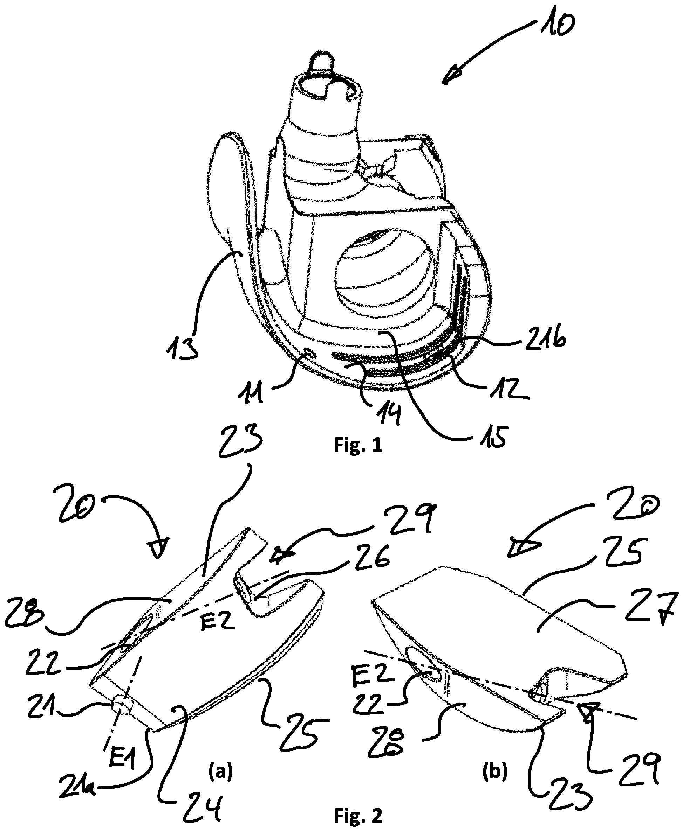

Die in Figur 1 veranschaulichte beispielhafte Ausführungsform weist als Gelenkkomponente 10 die Femurkomponente einer Knieendoprothese auf. Selbstverständlich ist das erfindungsgemäße Zwischensegment auch an anderen Gelenkkomponenten einsetzbar, die eine konkave Implantationsfläche aufweisen.

Am distalen Ende der in Figur 1 veranschaulichten beispielhaften Gelenkkomponente 10 ist eine konvexe Gelenkfläche 13 vorgesehen, die im implantierten Zustand des Gelenkersatzes der Gelenkfläche einer zweiten Gelenkkomponente zugewandt ist.

Auf der der Gelenkfläche 13 gegenüberliegenden Seite der Gelenkkomponente 10 ist eine konkave Implantationsfläche 14 vorgesehen. Diese konkave Implantationsfläche 14 ist im implantierten Zustand der Gelenkkomponente 10 dem Knochengewebe des Patienten zugewandt.

Im Normalfall ist die konkave Implantationsfläche 14 so relativ zu dem Knochengewebe eines Implantationsorts platzierbar, dass eine optimale Verbindung mit dem benachbarten Knochengewebe erreicht werden kann. Dies bedeutet zum Beispiel bei einer Verankerung der Gelenkkomponente 10 mittels Knochenzement, dass sich das Knochengewebe in einem vorbestimmten Abstand zu der Implantationsfläche 14 befindet, sodass der zur Befestigung dienende Knochenzement zwischen der konkaven Implantationsfläche 14 und dem Knochengewebe eine Verbindung herstellen kann. Ist die Gelenkkomponente 10 für eine Verankerung durch Einwachsen von Knochengewebe vorgesehen, so liegt das Knochengewebe des präparierten Implantationsorts bei Implantation möglichst direkt an der konkaven Implantationsfläche 14 an.

Wie oben erläutert gibt es allerdings Situationen, bei denen eine Positionierung der konkaven Implantationsfläche 14 relativ zu dem Knochengewebe des Patienten für eine optimale Verankerung der Gelenkkomponente 10 mangels ausreichendem Knochengewebe nicht möglich ist. Aus diesem Grund sind bei der Gelenkkomponente 10 in Figur 1 ein erstes Eingriffselement 11 und ein zweites Eingriffselement 12 vorgesehen.

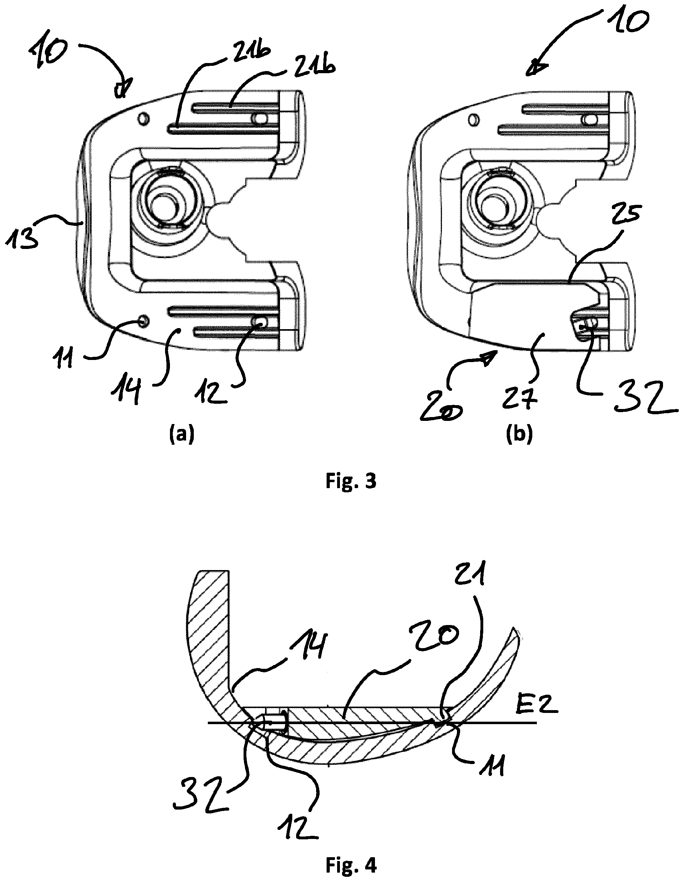

In diese Eingriffselemente 11, 12 greifen bei der beispielhaften Ausführungsform ein erstes Rastelement 21 bzw. ein zweites Rastelement 32 ein, um ein Zwischensegment 20 an der Gelenkkomponente 10 zu befestigen (siehe Figur 4). Neben mindestens zwei Rastelementen 11, 12 weist ein erfindungsgemäßes Zwischensegment 20 insbesondere einen Zwischensegmentkörper 23 auf.

Wie in den Figuren 2a und 2b veranschaulicht, ist an dem Zwischensegmentkörper 23 des Zwischensegments 20 eine zu der konkaven Implantationsfläche 14 der Gelenkkomponente 10 korrespondierende konvexe Seite 24 ausgebildet. Wie in Figur 4 zu erkennen ist, ist die konvexe Seite 24 des Zwischensegments 20 vorzugsweise dafür vorgesehen, an der konkaven Implantationsfläche 14 der Gelenkkomponente 10 anzuliegen. Diesbezüglich ist zu beachten, dass je größer die anliegende Fläche der konvexen Seite 24 des Zwischensegmentkörpers 23 ist, desto besser ist eine Übertragung von Gelenkkräften über das zwischen dem Knochengewebe und der Gelenkkomponente 10 angeordnete Zwischensegment 20 möglich. Dementsprechend liegt vorzugsweise die gesamte der konkaven Implantationsfläche 14 zugewandte konvexe Seite 24 des Zwischensegmentkörpers 23 an der Implantationsfläche 14 an. Die konvexe Seite des Zwischensegmentkörpers 23 und/oder die konkave Implantationsfläche 14 der Gelenkkomponente 10 kann dabei ein gekrümmtes und/oder facettenartiges Profil aufweisen.

Weiterhin kann der Zwischensegmentkörper 23, wie in der beispielhaften Ausführungsform der Figuren 2a und 2b veranschaulicht, eine weitere der Gelenkkomponente 10 zugewandte Seite 25 aufweisen. Diese liegt im montierten Zustand der Seite 15 der Gelenkkomponente 10 gegenüber (siehe Figur 1). Eine dritte der Gelenkkomponente 10 zugewandte Seite 26 des Zwischensegmentkörpers 23 wird nachfolgend noch genauer erläutert.

Die Rastelemente 21, 32 sind auf mindestens einer Seite 24, 25, 26 des Zwischensegments 20 angeordnet, die jeweils der Gelenkkomponente 10 zugewandt sind. Auf diese Weise sind die Rastelemente 21, 32 mit dem Gelenkkörper 10 in Eingriff bringbar. Bei der in Figur 2 veranschaulichten beispielhaften Ausführungsform ist ein feststehendes Rastelement 21 an der konvexen Seite 24 des Zwischensegmentkörpers 23 und ein bewegliches Rastelement 32 an der dritten der Gelenkkomponente 10 zugewandten Seite 26 des Zwischensegmentkörpers 23 angeordnet. Allerdings ist auch eine Anordnung der Rastelemente 21, 32 bei dem Zwischensegment auch an einer anderen der Gelenkkomponente 10 zugewandten Seite möglich. So ist zum Beispiel in Bezug auf Figur 1 denkbar, dass ein Rastelement 21, 32 des Zwischensegments 20 mit einem entsprechenden Eingriffselement 11, 12 auf der Seite 15 der Gelenkkomponente 10 in Eingriff bringbar ist.

Wie bei der in den Figuren gezeigten beispielhaften Ausführungsform des Zwischensegments 20 gezeigt, ist das feststehende Rastelement 21 vorzugsweise als Vorsprung ausgebildet. Gleiches gilt für das bewegliche Rastelement 32.

Das feststehende erste Rastelement 21 kann weiterhin, wie in den Figuren 2a und 4 gezeigt, integral mit dem Zwischensegmentkörper 23 ausgebildet sein. Dies hat den Vorteil, dass es eine besonders hohe strukturelle Festigkeit aufweist. Weiterhin ist das in Figur 2a gezeigte feststehende Rastelement 21 in dieser beispielhaften Ausführungsform als Zylinder ausgebildet. Dieser Zylinder weist eine Eingriffsachse E1 auf, über die das feststehende Rastelement 21 des Zwischensegments 20 mit dem Eingriffselement 11 der Gelenkkomponente 10 in Eingriff bringbar ist.

Es ist verständlich, dass das Rastelement 21 eine beliebige Form aufweisen kann, solange diese einen Eingriff mit der Gelenkkomponente 10 ermöglicht. Beispielsweise ist es möglich, eine Kante des Zwischensegmentkörpers 23, wie zum Beispiel die in Figur 2a mit dem Bezugszeichen 21a gekennzeichnete Kante, für einen Eingriff mit der Gelenkkomponente 10 vorzusehen. Eine solche Eingriffskante 21a könnte mit einer entsprechenden Aussparung in der konkaven Implantationsfläche 14 in Eingriff gehen.

Weiterhin kann ein Rastelement als Nut oder als länglicher Vorsprung ausgeführt sein. Nuten mit solch einer Funktion können zum Beispiel so wie die in der Implantationsfläche 14 ausgebildeten Nuten 21b ausgeführt sein. Es ist jedoch darauf hinzuweisen, dass die Nuten 21b bei der veranschaulichten Ausführungsform keine derartige Funktion aufweisen. Dies ist an der konvexen Seite 24 des Zwischensegmentkörpers 23 leicht zu erkennen, da dieser keine entsprechenden Rastelemente aufweist. Zudem kann auch das erste Rastelement 21 wie das zweite Rastelement 32 als bewegliches Rastelement ausgeführt sein.

Weiterhin weist das Zwischensegment 20 ein bewegliches Rastelement 32 auf, das mit dem zweiten Eingriffselement 12 der Gelenkkomponente 10 in Eingriff bringbar ist. Bei der hier gezeigten bevorzugten Ausführungsform des Zwischensegments 20 ist das bewegliche Rastelement 32 als Stift ausgeführt. Das bewegliche Rastelement 32 weist in Längsrichtung an einem seiner Enden einen Verriegelungsabschnitt und an dem entgegengesetzten Ende vorzugsweise einen nicht gezeigten Werkzeugeingriffsabschnitt auf. Wie bei der in den Figuren 3b und 4 gezeigten beispielhaften Ausführungsform erkennbar ist, kann der Verriegelungsabschnitt sich verjüngend und insbesondere konisch ausgeführt sein, um einen Eingriff in das Eingriffselement 12 zu erleichtern.

Das bewegliche Rastelement 32 ist entlang einer zweiten Eingriffsachse E2 von einer Freigabeposition zu einer Verriegelungsposition bewegbar. In der Verriegelungsposition greift das Rastelement 32, wie in Figur 4 gezeigt, in das Eingriffselement 12 der Gelenkkomponente 10 ein. Die Bewegung des Rastelements 32 wird bei dem veranschaulichten Ausführungsbeispiel durch einen nicht gezeigten Gewindeeingriff ermöglicht. Hierfür weist das Rastelement 32 ein nicht gezeigtes Außengewinde auf. Dieses Außengewinde greift in ein Innengewinde ein, das in einem Durchgangsloch 22 des Zwischensegmentkörpers 23 vorgesehen ist. Wie in Figur 2 gezeigt, befindet sich eine Öffnung des Durchgangslochs 22 vorzugsweise auf einer freien Seite 28 des Zwischensegmentkörpers 23. Dadurch ist es möglich, ein Werkzeug selbst dann über diese Öffnung mit dem Werkzeugeingriffsabschnitt des Rastelements 32 in Eingriff zu bringen, wenn die Gelenkkomponente 10 bereits implantiert ist. Dies hat insbesondere die oben im Zusammenhang mit einer Revisionsoperation beschriebenen Vorteile.

Die zweite Öffnung des Durchgangslochs 22 befindet sich bei dem in Figur 2 veranschaulichten Ausführungsbeispiel in der dritten der Gelenkkomponente 10 zugewandten Seite 26 des Zwischensegmentkörpers 23. Die dritte Seite 26 des Zwischensegmentkörpers 23 bildet eine Aussparung 29 aus, über die ein Eingriff des beweglichen Rastelements 32 in das entsprechende Eingriffselement 12 der Gelenkkomponente 10 beobachtet und überprüft werden kann (vergleiche Figur 3b).

Weiterhin kann die im implantierten Zustand dem Knochengewebe zugewandte Seite des Zwischensegmentkörpers 23 als Verankerungsfläche 27 ausgeführt sein. Dabei wird die Verankerungsfläche 27 mit einer Oberfläche bereitgestellt, welche entsprechend der gewählten Verankerungstechnik einer Verankerung bzw. Verbindung des Knochengewebes mit dem Zwischensegment 20 und damit der Gelenkkomponente 10 fördert. Für eine Implantation geeignete Verankerungstechniken sind insbesondere eine Verbindung über Knochenzement oder eine Verankerung über Knocheneinwuchs in die Oberfläche des Implantats. Folglich kann durch die weiter oben bereits beschriebenen Ausführungen der Verankerungsfläche 27 das Zwischensegment die Verankerung der Gelenkkomponente 10 unterstützen und so die Funktion der Implantationsfläche übernehmen.

Für die Montage eines Zwischensegments 20 an einer Gelenkkomponente 10 wird zunächst ein Zwischensegment 20 ausgewählt, dessen Zwischensegmentkörper 23 eine möglichst gute Anpassung der Geometrie der Gelenkkomponente 10 an den Implantationsort ermöglicht. Mit anderen Worten kann der Zwischensegmentkörper 23 im besten Fall das fehlende Knochengewebe ersetzen.

Das ausgewählte Zwischensegment 20 wird anschließend über die mindestens zwei Rastelemente 21, 22 mit dem ersten Eingriffselement 11 bzw. dem zweiten Eingriffselement 12 der konkaven Implantationsfläche 14 in Eingriff gebracht und dadurch an der Gelenkkomponente 10 verriegelt.

Bei der in Figur 2 gezeigten beispielhaften Ausführungsform eines Zwischensegments 20 erfolgt die Verriegelung des Zwischensegments 20 an der Gelenkkomponente 10, indem zunächst das feststehende Rastelement 21 mit dem entsprechenden Eingriffselement 11 an der konkaven Implantationsfläche 14 der Gelenkkomponente 10 in Eingriff gebracht wird. Der Eingriff erfolgt dabei entlang einer ersten Eingriffsachse E1.

Als Nächstes wird das bewegliche Rastelement 32 mit dem zweiten Eingriffselement 12 über eine zweite Eingriffsachse E2 in Eingriff gebracht. Dies erfolgt bei der beispielhaften Ausführungsform durch Einführen eines Werkzeugs in das Durchgangsloch 22 und in Eingriff Bringen der Werkzeugspitze mit einem Werkzeugeingriffsabschnitt des beweglichen Rastelements 32.

Bei der in den Figuren veranschaulichten Ausführungsform wird das als Stift ausgeführte Rastelement 32 über das Werkzeug um seine Längsachse gedreht, sodass es über einen Gewindeeingriff mit dem Zwischensegmentkörper 23 in seiner Längsrichtung zwischen einer Freigabeposition, bei der das Zwischensegment 20 von der Gelenkkomponente 10 lösbar ist, und einer Verriegelungsposition, bei der das Zwischensegment mit der Gelenkkomponente verriegelt ist, bewegbar ist. Zudem kann das Gewinde selbsthemmend ausgeführt sein, sodass sich das bewegliche Rastelement nicht unbeabsichtigt von der Verriegelungsposition zu der Freigabeposition bewegen kann.

Diese so ermöglichte Bewegung des Rastelements 32 zwischen einer Freigabeposition und einer Verriegelungsposition ist vorzugsweise über einen Sichtabschnitt, der durch eine Aussparung 29 ausgebildet wird, welche wiederum zumindest teilweise von der Seite 26 ausgebildet wird, für den Benutzer sichtbar, sodass eine korrekte Verriegelung sichergestellt werden kann.

Anstelle eines als Stift ausgeführten Rastelements 32 kann dieses auch hakenförmig ausgebildet sein und über eine Drehbewegung mit der Gelenkkomponente 10 in Eingriff gebracht werden.

Unabhängig von dem Mechanismus, der das bewegliche Rastelement 32 in Eingriff bringt, erfolgt eine Verriegelung des Zwischensegments 20 an der Gelenkkomponente 10 vorzugsweise über einen Formschluss. D. h., dass die Rastelemente 21, 32 so mit den Eingriffselementen 11, 12 der Gelenkkomponente 10 in Eingriff bringbar sind, dass das Zwischensegment 23 an der Gelenkkomponente 10 verriegelt ist. Im verriegelten Zustand sind die Rastelemente 21, 32 und Eingriffselemente 11, 12 so im Eingriff, dass das Zwischensegment 20 in keiner Richtung mehr von der Gelenkkomponente 10 abgezogen werden kann. Mit anderen Worten weist das Zwischensegment 20 im verriegelten Zustand relativ zu der Gelenkkomponente 10 keinen Freiheitsgrad auf.

Insbesondere bei einem Formschluss ist das Zwischensegment vorzugsweise aus Metall ausgeführt, um so eine Verriegelung mit einer hohen Festigkeit bereitzustellen.

Somit stellt das Zwischensegment der vorliegenden Erfindung eine kostengünstige und einfach handhabbare Lösung bereit, mit der eine Gelenkkomponente 10 auch bei unzureichend vorhandenem Knochengewebe zuverlässig an diesem verankert werden kann und gleichzeitig gegen eine weitere Verletzung des Knochengewebes vorbeugt.

### BEZUGSZEICHEN

- 10: Gelenkkomponente
- 11: erstes Eingriffselement
- 12: zweites Eingriffselement
- 13: konvexe Gelenkfläche
- 14: konkave Implantationsfläche
- 15: dem Zwischensegment zugewandte Seite
- 20: Zwischensegment
- 21: erstes Rastelement (feststehendes Rastelement)
- 21a: als Rastelement verwendbare Kante
- 21b: als Rastelement verwendbare Nut oder Vorsprung
- 22: Durchgangsloch mit Innengewinde
- 23: Zwischensegmentkörper
- 24: erste der Gelenkkomponente zugewandte Seite (konvexe Seite)
- 25: zweite der Gelenkkomponente zugewandte Seite
- 26: dritte der Gelenkkomponente zugewandte Seite
- 27: Verankerungsfläche
- 28: freie Seite des Zwischensegmentkörpers
- 29: Aussparung am Zwischensegmentkörper
- 32: zweites Rastelement (bewegliches Rastelement)

- E1: erste Eingriffsachse
- E2: zweite Eingriffsachse

## Patentansprüche

1. Zwischensegment (20) für eine Anordnung zwischen einer konkaven Implantationsfläche (14) einer Gelenkkomponente (10), und Knochengewebe, wobei das Zwischensegment (20) einen Zwischensegmentkörper (23) mit mindestens einer der Gelenkkomponente (10) zuzuwendenden Seite (24, 25) aufweist, wobei eine (24) der mindestens einen der Gelenkkomponente zuzuwendenden Seite konvex ist, und **dadurch gekennzeichnet, dass** das Zwischensegment (20)einen Verriegelungsmechanismus zum Anbringen des Zwischensegments an der Gelenkkomponente mit mindestens einem ersten (21) und einem zweiten (32) Rastelement aufweist, wobei mindestens eines der Rastelemente (21, 32) beweglich ist und das erste Rastelement (21) eine erste Eingriffsachse (El) und das zweite Rastelement (32) eine zweite Eingriffsachse (E2) aufweist, wobei der Winkel zwischen der ersten Eingriffsachse und der zweiten Eingriffsachse ungleich null ist.

2. Zwischensegment (20) nach Anspruch 1, bei dem das mindestens eine bewegliche Rastelement (32) zwischen einer Freigabeposition und einer Verriegelungsposition bewegbar ist und bei dem das bewegliche Rastelement (32) in der Verriegelungsposition vorzugsweise von dem Zwischensegmentkörper (23) hervorsteht.

3. Zwischensegment (20) nach Anspruch 1 oder 2, bei dem eines der Rastelemente (21, 32) ein feststehendes Rastelement (21) ist, wobei das feststehende Rastelement (21) vorzugsweise von dem Zwischensegmentkörper (23) hervorsteht.

4. Zwischensegment (20) nach einem der vorstehenden Ansprüche, bei dem der Zwischensegmentkörper (23) mindestens eine Verankerungsfläche (27) zum Anbringen an Knochengewebe aufweist, wobei die Verankerungsfläche (27) vorzugsweise eine Oberflächenstruktur und/oder Beschichtung aufweist, die noch bevorzugter für ein Einwachsen von Knochengewebe eingerichtet ist.

5. Zwischensegment (20) nach einem der vorstehenden Ansprüche, bei dem die Rastelemente (21, 32) zum Zusammenwirken mit der Gelenkkomponente (10) an der konvexen Seite (24) des Zwischensegments (20) angeordnet sind.

6. Zwischensegment (20) nach einem der vorstehenden Ansprüche, bei dem das bewegliche Rastelement (32) als Stift ausgebildet ist, der in seiner Längsrichtung auf einer Seite einen Verriegelungsabschnitt und auf der anderen gegenüberliegenden Seite vorzugsweise einen Werkzeugeingriffsabschnitt aufweist und noch bevorzugter als Gewindestift ausgebildet ist.

7. Zwischensegment (20) nach einem der Ansprüche 1 bis **5,**
bei dem das bewegliche Rastelement (32) als Stift ausgebildet ist, der in seiner Längsrichtung auf einer Seite einen Verriegelungsabschnitt und auf der anderen gegenüberliegenden Seite einen Werkzeugeingriffsabschnitt aufweist und bevorzugt als Gewindestift ausgebildet ist, und
bei dem der Werkzeugeingriffsabschnitt des Stifts für ein Bewegen des Stifts von einer freien Seite des Zwischensegmentkörpers (23) aus zugänglich ist.

8. Zwischensegment (20) nach einem der vorstehenden Ansprüche, bei dem der Zwischensegmentkörper (23) einen bevorzugt als Aussparung (29) ausgeführten Sichtabschnitt aufweist.

9. Gelenkkomponente (10) mit einer konkaven Implantationsfläche (12) mit einem über einen Verriegelungsmechanismus an der Gelenkkomponente (10) befestigbaren Zwischensegment (20) nach einem der vorstehenden Ansprüche.

10. Gelenkkomponente (10) nach Anspruch 9 mit mindestens zwei Eingriffselementen (11, 12), wobei die Eingriffselemente (11, 12) auf zumindest einer dem Zwischensegment (20) zugewandten Seite (14, 15), vorzugsweise der konkaven Implantationsfläche (14), angeordnet sind.

11. Gelenkkomponente (10) nach Anspruch 9 oder 10, bei der das Zwischensegment (20) über einen Formschluss an der Gelenkkomponente (10) befestigbar ist.

12. Gelenkkomponente (10) nach einem der Ansprüche 9 bis 11, wobei die Gelenkkomponente eine Femurprothese ist.

13. Verfahren zum Montieren und Befestigen eines Zwischensegments (20) nach einem der Ansprüche 1 bis 8, an einer konkaven Implantationsfläche (14) einer Gelenkkomponente (10) vor Implantation der Gelenkkomponente, wobei das Verfahren die Schritte umfasst:
- in Eingriff Bringen eines ersten Rastelements (21) des Zwischensegments (20) mit einem ersten Eingriffselement (11) der Gelenkkomponente (10),
- in Eingriff Bringen eines zweiten Rastelements (32) des Zwischensegments (20) mit einem zweiten Eingriffselement (12) der Gelenkkomponente (10),
wobei mindestens eines der Rastelemente des Zwischensegments ein bewegliches Rastelement ist und die Eingriffsachsen (El, E2) der Rastelemente (21, 32) einen Winkel aufweisen, der ungleich null ist.

## Claims

1. An intermediate segment (20) for an arrangement between a concave implantation surface (14) of a joint component (10) and bone tissue, wherein the intermediate segment (20) comprises an intermediate segment body (23) having at least one side (24, 25) to be turned toward the joint component (10), wherein a side (24) to be turned toward the at least one joint component is convex, **characterized in that** the intermediate segment (20) comprises a locking mechanism for attaching the intermediate segment to the joint component having at least one first (21) and one second (32) detent element, wherein at least one of the detent elements (21, 32) is movable and the first detent element (21) comprises a first engagement axis (E1) and the second detent element (32) comprises a second engagement axis (E2), wherein the angle between the first engagement axis and the second engagement axis is not equal to zero.

2. The intermediate segment (20) as claimed in claim 1, in which the at least one movable detent element (32) is movable between a release position and a locking position and in which the movable detent element (32) preferably protrudes from the intermediate segment body (23) in the locking position.

3. The intermediate segment (20) as claimed in claim 1 or 2, in which one of the detent elements (21, 32) is a fixed detent element (21), wherein the fixed detent element (21) preferably protrudes from the intermediate segment body (23).

4. The intermediate segment (20) as claimed in any one of the preceding claims, in which the intermediate segment body (23) comprises at least one anchoring surface (27) for attachment to bone tissue, wherein the anchoring surface (27) preferably comprises a surface structure and/or coating, which is still more preferably configured for an ingrowth of bone tissue.

5. The intermediate segment (20) as claimed in any one of the preceding claims, in which the detent elements (21, 32) for interaction with the joint component (10) are arranged on the convex side (24) of the intermediate segment (20).

6. The intermediate segment (20) as claimed in any one of the preceding claims, in which the movable detent element (32) is designed as a pin, which, in its longitudinal direction, comprises a locking section on one side and preferably comprises a tool engagement section on the other opposing side and still more preferably is designed as a threaded pin.

7. The intermediate segment (20) as claimed in any one of claims 1 to 5,
in which the movable detent element (32) is designed as a pin, which, in its longitudinal direction, comprises a locking section on one side and comprises a tool engagement section on the other opposing side and is preferably designed as a threaded pin, and
in which the tool engagement section of the pin is accessible from a free side of the intermediate segment body (23) for a movement of the pin.

8. The intermediate segment (20) as claimed in any one of the preceding claims, in which the intermediate segment body (23) comprises a viewing section preferably embodied as a recess (29).

9. A joint component (10) having a concave implantation surface (12) having an intermediate segment (20) as claimed in any one of the preceding claims, which is fastenable via a locking mechanism on the joint component (10).

10. The joint component (10) as claimed in claim 9 having at least two engagement elements (11, 12), wherein the engagement elements (11, 12) are arranged on at least one side (14, 15) facing toward the intermediate segment (20), preferably the concave implantation surface (14).

11. The joint component (10) as claimed in claim 9 or 10, in which the intermediate segment (20) is fastenable via a form fit on the joint component (10).

12. The joint component (10) as claimed in any one of claims 9 to 11, wherein the joint component is a femur prosthesis.

13. A method for installing and fastening an intermediate segment (20) as claimed in any one of claims 1 to 8 on a concave implantation surface (14) of a joint component (10) before implantation of the joint component, wherein the method comprises the following steps:
- engaging a first detent element (21) of the intermediate segment (20) with a first engagement element (11) of the joint component (10),
- engaging a second detent element (32) of the intermediate segment (20) with a second engagement element (12) of the joint component (10),
wherein at least one of the detent elements of the intermediate segment is a movable detent element and the engagement axis (E1, E2) of the detent elements (21, 32) have an angle which is not equal to zero.

## Revendications

1. Segment intermédiaire (20) pour un agencement entre une surface d'implantation concave (14) d'un composant d'articulation (10) et du tissu osseux, dans lequel le segment intermédiaire (20) présente un corps de segment intermédiaire (23) avec au moins un côté (24, 25) orienté vers le composant d'articulation (10), dans lequel l'un (24) de l'au moins un côté orienté vers le composant de joint est convexe, et **caractérisé en ce que** le segment intermédiaire (20) présente un mécanisme de verrouillage pour fixer le segment intermédiaire au composant d'articulation avec au moins un premier (21) et un second (32) élément de verrouillage, dans lequel au moins l'un des éléments de verrouillage (21, 32) est mobile et le premier élément de verrouillage (21) présente un premier axe de mise en prise (E1) et le second élément de verrouillage (32) présente un second axe de mise en prise (E2), dans lequel l'angle entre le premier axe de mise en prise et le second axe de mise en prise est non nul.

2. Segment intermédiaire (20) selon la revendication 1, dans lequel l'au moins un élément de verrouillage mobile (32) est mobile entre une position de libération et une position de verrouillage et dans lequel l'élément de verrouillage mobile (32), en position de verrouillage, fait saillie de préférence depuis le corps de segment intermédiaire (23).

3. Segment intermédiaire (20) selon la revendication 1 ou 2, dans lequel l'un des éléments de verrouillage (21, 32) est un élément de verrouillage fixe (21), dans lequel l'élément de verrouillage fixe (21) fait saillie de préférence depuis le corps de segment intermédiaire (23).

4. Segment intermédiaire (20) selon l'une des revendications précédentes, dans lequel le corps de segment intermédiaire (23) présente au moins une surface d'ancrage (27) pour la fixation au tissu osseux, dans lequel la surface d'ancrage (27) présente de préférence une structure superficielle et/ou un revêtement qui est de préférence adapté à la croissance interne du tissu osseux.

5. Segment intermédiaire (20) selon l'une des revendications précédentes, dans lequel les éléments de verrouillage (21, 32) sont disposés sur le côté convexe (24) du segment intermédiaire (20) pour une coopération avec le composant d'articulation (10).

6. Segment intermédiaire (20) selon l'une des revendications précédentes, dans lequel l'élément de verrouillage mobile (32) est réalisé sous la forme d'une goupille qui, dans sa direction longitudinale, présente une section de verrouillage d'un côté et de préférence une section de mise en prise d'outil de l'autre côté opposé, et de manière davantage préférée encore, est conçue comme une vis de réglage.

7. Segment intermédiaire (20) selon l'une des revendications 1 à 5,
dans lequel l'élément de verrouillage mobile (32) est conçu comme une tige qui présente, d'un côté, dans sa direction longitudinale, une section de verrouillage, et de l'autre côté opposé, une section de mise en prise d'outil, et est de préférence conçu comme une tige filetée, et
dans lequel la section de mise en prise d'outil de la broche est accessible pour déplacer la broche depuis un côté libre du corps de segment intermédiaire (23).

8. Segment intermédiaire (20) selon l'une des revendications précédentes, dans lequel le corps de segment intermédiaire (23) présente une section visible, de préférence réalisée sous forme d'évidement (29).

9. Composant d'articulation (10) avec une surface d'implantation concave (12) avec un segment intermédiaire (20) qui peut être fixé au composant d'articulation (10) par l'intermédiaire d'un mécanisme de verrouillage selon l'une des revendications précédentes.

10. Composant d'articulation (10) selon la revendication 9, comportant au moins deux éléments de mise en prise (11, 12), dans lequel les éléments de mise en prise (11, 12) sont disposés sur au moins un côté (14, 15) orienté vers le segment intermédiaire (20), de préférence la surface d'implantation concave (14).

11. Composant d'articulation (10) selon la revendication 9 ou 10, dans lequel le segment intermédiaire (20) peut être fixé au composant d'articulation (10) par complémentarité de forme.

12. Composant d'articulation (10) selon l'une quelconque des revendications 9 à 11, dans lequel le composant d'articulation est une prothèse fémorale.

13. Procédé de montage et de fixation d'un segment intermédiaire (20) selon l'une des revendications 1 à 8, sur une surface concave d'implantation (14) d'un composant d'articulation (10) avant implantation du composant d'articulation, dans lequel le procédé comprend les étapes :
- de mise en prise d'un premier élément de verrouillage (21) du segment intermédiaire (20) avec un premier élément de mise en prise (11) du composant d'articulation (10),
- de mise en prise d'un second élément de verrouillage (32) du segment intermédiaire (20) avec un second élément de mise en prise (12) du composant d'articulation (10),
dans lequel au moins l'un des éléments de verrouillage du segment intermédiaire est un élément de verrouillage mobile et les axes de mise en prise (E1, E2) des éléments de verrouillage (21, 32) présentent un angle qui n'est pas égal à zéro.
